# EUROPEAN PATENT SPECIFICATION

(11) **EP 1 713 449 B1**
(45) Date of publication and mention of the grant of the patent: **09.03.2011**
(21) Application number: 05711993.5
(22) Date of filing: 25.01.2005
(51) Int. Cl.: A61K 8/02, A61K 8/11, A61Q 19/00

(54) **NONABRASIVE SENSORY EXFOLIATING SYSTEM**
SCHONENDES, DIE SINNE BELEBENDES PEELING-SYSTEM
SYSTEME D'EXFOLIATION NON ABRASIF SENSORIEL

(30) Priority: 30.01.2004 US 540562 P
(43) Date of publication of application: 25.10.2006
(73) Proprietor: E-L Management Corp., New York, NY 10153 (US)
(72) Inventor: MOHAMMADI, Fatemeh, Hauppauge, New York 11788 (US); CZARNOTA, Anna, Huntington, New York 11746 (US); HARRISON, James, Blue Point, New York 11715 (US); LEONARD, Carin, Uniondale, New York 11553 (US)
(74) Representative: Hirsch & Associés
(86) International application number: PCT/US2005/002328
(87) International publication number: WO 2005/074509

(56) References cited:
- EP-A- 0 688 561
- US-A- 3 250 680
- US-A- 5 879 797
- US-A1- 2003 108 506
- US-A1- 2003 211 062

## Description

### Field of the Invention

The present invention relates to cosmetic compositions containing a methods of exfoliating the skin with a set of diversely sized particulate system in a sensory heating mask that has a substantially nonabrasive-feeling and heating sensation on the skin. In particular, the invention relates to topically applied compositions containing a combination of a heat generating agent and a physical exfoliating system of diversely sized particles.

### Background of the Invention

To exfoliate the epidermis, compositions have been proposed in the form of creams containing abrasive substances comprised of insoluble particles in the appropriate size and shape such as, for example, quartz particles or finely ground shells, nuts or grains which, after application to the parts of the body to be cleansed and exfoliated, are removed by wiping or rinsing with water. Such compositions are particularly effective but may be irritating to the skin because they can be too abrasive. In addition, particles of the abrasive materials can remain in the pores of the skin and thus can cause clogged pores which can in turn incite skin conditions such as blackheads and acne.

The cells of the outermost layer of the stratum corneum are constantly shed naturally, by the normal process of desquamation, as minute particles. When fully keratinised tissue loses its cellular structure and reaches the surface of the stratum corneum, it breaks up into microscopic squames and sheds off the surface of the skin. Microscopic squames at the skin surface are commonly referred to as dead skin cells and make up a dead skin layer on the skin surface. The process of desquamation has been estimated to cause a loss of tissue in an amount of up to 14 grams per day. This loss is constantly replenished with cells from lower layers of the epidermis. Thus, the layers of the epidermis are composed of cells moving up towards the surface in successive stages of differentiation until death when they are finally sloughed off and lost to the environment. Desquamation is one of the processes by which skin maintains its health and vitality as nutrients and moisture are continuously replaced on the surface of the skin when dead skin cells are removed. Normally, the desquamation process takes about 14 days (i.e., the corneocyte takes 14 days to reach the outermost layer of the strateum corneum to be shed). When desquamation does not take place regularly, the surface of the skin tends to become rough and flaky, and wrinkles as well as other undesirable effects of skin aging may appear on the surface of the skin. To improve these skin conditions, in addition to or as an alternative to the natural desquamation process, exfoliation is often used to rejuvenate and enhance the health of the skin at any age.

Exfoliation is a technique whereby dead skin cells are removed or sloughed from the skin surface to promote a healthier and more youthful appearance to the skin. There are physical exfoliants such as the quartz and the finely ground shells, nuts and grains, previously noted above, and there are chemical exfoliants. Jojoba meal has been used in several instances, for example, a lotus and jade line of products by Pulanna, a body exfoliator is part of a line of products by Healing Garden Spa Therapy, and bamboo scrub is available from Raya. Further these ingredients are disclosed in patents such as JP 1226806 and U.S. Patent No. 5,879,797. Several compounds are known to be useful as chemical exfoliants such as for example, alpha hydroxy acid ("AHA"), beta hydroxy acid ("BHA"), retinoic acid ("retin A"), and enzymes. An exfoliant such as AHA breaks the bond holding individual squames together and allows them to detach and shed. These types of exfoliants are considered to be chemically based rather than physically based. The distinguishing feature between the two is that the chemically based exfoliant acts on the bond of the squames, whereas the physically based exfoliant acts to physically slough off the layer of dead skin cells where the bond of the squames has already broken down.

US 2003/0211062 relates to anhydrous skin cleansers and polishing formulations. The disclosed size of particules is in the range of about 50 to 1000 microns. During the use of the cleanser, "the abrasive particles begin to dissolve and the particule size becomes smaller" so that it "acts as a finer and finer abrasive agent that assists in smoothing, polishing and buffing the now cleansed surface of the skin". There is neither disclosure of three different types of exfoliating particles nor of the use of a silicon vehicule or carrier for encapsulating a heat generating agent, for providing a water-insoluble exfoliating material.

Producing a variety of alternatives for exfoliation is desirable because skin types vary among consumers, and therefore, making a variety of exfoliants available to meet various skincare needs of individual consumers is beneficial. Thus, there is a continued effort to find alternative ways of aiding the sloughing ability of the skin and promoting its health for various types of skin. Particularly, there is a need to improve physical exfoliation systems from clogging the pores of the skin. This is, therefore, an object of the present invention.

### Summary of the Invention

This invention relates to exfoliating compositions and methods whereby an encapsulated heating agent and a set of diversely sized particles are applied to the skin for microdermabrasion. These compositions have the added benefit of feeling substantially nonabrasive on the skin. Specifically, the exfoliating compositions and methods of the present invention comprises a heating agent, a silicone component, and a set of diversely sized particles. Upon application to the skin, a warm and soothing microdermabrasion affect is felt on the skin surface. These compositions have the added benefit of feeling substantially nonabrasive on the skin. Thus, the methods provide a smooth, substantially nonabrasive and warm feeling to the exfoliation process and the exfoliating composition rinses off of the skin easily.
The physical exfoliating system of diversely sized particles has at least three insoluble particles of different types of materials. Each of the three materials has a particle size that is at least 50 microns different than the other two materials. Because of the varying sizes of the particles in the exfoliating system and the generation of heat energy by the heat-generating agent, the physical exfoliants do not settle into the pores of the skin and feel less abrasive on the skin surface. The method of exfoliating the skin is achieved using, preferably, a cosmetic mask that is applied to the facial skin surface. The mask provides a heating sensation that soothes while it nourishes, purifies, detoxifies, and remineralizes the skin surface in the exfoliation process. The skin surface after treatment with the mask has improved clarity and smoothness.

### Detailed Description of the Invention

It has now been unexpectedly discovered that a physical exfoliating system that is topically applied to the skin is substantially less abrasive and rinses off easily when it is applied in combination with a heat generating agent and a silicone component. The heating agent can be any type of self contained heat generating means such as those described in U.S. Patent No. 3,240,396 and 3,723,324. Examples of heating agents, include but are not limited to, metallic chlorides, bromides, oxides and hydroxides, carbonates and sulfates. The particle size of the heating agent is preferably of a small particle size such as for example, 50 microns or less. In a preferred embodiment of the present invention, the heating agent is encapsulated by any known means of encapsulation including, but not limited to, for example, surface treatment with a silicone. Preferably, the heating agent is encapsulated with a combination of silica and dimethicone fluid and is available under the commercial name Natural Hot^{™} TR by Resources of Nature, Inc., South Plainfield, New Jersey. The amount of the encapsulated heating agent can be 10 to 40 percent by weight of the composition, and preferably up to about 35 percent by weight of the composition. When the compositions of the present invention are in the form of a mask, the heat sensation that is felt on the skin surface is optimized.

The physical exfoliating system has particles of at least three exfoliating materials of varying sizes. Particles useful as exfoliants are well known in the art and any type of particle can be used. Examples include, but are not limited to, olive stone, jojoba meal, bran, wheat flour grains, almond meal, corn meal, oatmeal, walnut shell powder, ground bamboo, jade powder, acetal resins, aluminum oxide, boron carbide, calcium carbonate, calcium phosphate, calcium silicate, diatomaceous earth, resins of polyamide, polyethylene, polytetrafluoroethylene, polypropyene, polyurethane, silica, pumice, quartz, silicon nitride, silicon carbide, titanium dioxide, and other ground wood. The particle size of the set of exfoliating particles is in the range of between about 20 to 500 microns and each particle having a certain average particle size has a particle size difference of at least 50 microns from the other particles in the set. Specifically, the largest particles have a particle size in the range of between about 300 to 500 microns; the smallest particles have a particle size in the range of between about 20 to 50 microns; and, the third group of particles in the middle has a particle size in the range of between about 50 to 300 microns. Preferably, the largest particle is a bamboo extract, the middle-sized particle is polyethylene resin and the smallest particle is a jade powder.

The smallest particle of jade powder is known for use in cosmetics and personal care products. The jade powder has an INCI name of nephrite powder and is prepared by a cryogenic particle size reduction technology that produces a fine, velvety powder that is easily incorporated into creams and lotions. The particle size of the jade powder is about 90 percent min through a 400 mesh (about 38 µ).

The silicone component comprises a variety of silicone based materials including but not limited to, for example, silicone fluids, both volatile and non-volatile; silicone copolymers, including silicone gums; and silicone cross-linked polymers. Examples of silicone fluids include, but are not limited to, any methylated linear or cyclic non-elastomeric organopolysiloxane, or combinations thereof. Preferably, however, the vehicle is a low-volatile silicone oil, such as dimethicone, penta cyclosiloxane (D5), or a mixture of such oils. Particularly preferred is a low viscosity, low-volatile silicone, for example, a 20cs dimethicone. Examples of suitable volatile silicones oils include cyclic and linear silicones, such as cyclomethicone, octamethylcyclotetrasiloxane, and decamethylcyclopentasiloxane. Particularly preferred, however, are non-volatile cyclic silicones, such as dimethicones having a viscosity of greater that 10 centistokes, alkylated dimethicones, such as cetyl or stearyl dimethicone, and trimethicones, such as phenyl trimethicone. The silicone component provides an aesthetically pleasing vehicle for the composition of the present invention and can also enhance the exfoliating action of the diversely sized exfoliating particles.

Specifically, the silicone cross-linked polymers of the present invention are a reaction product of an organopolysiloxane having an unsaturated group bound to a terminal silicon atom and an organohydrogensiloxane which reaction product is at least partially cross-linked. Cross-links are junctions of polymer strands in a three-dimensional structured network. They are like long-chain branches which are so numerous that a continuous insoluble gel is formed. An elastomer is generally, a chain polymer having a degree of cross-linking sufficient to provide a gel-like substance. Such an elastomer may have a viscosity of about 100,000 to 1,000,000 cps. The elastomer can be cured by mechanisms known in the art such as addition-type or condensation-type.

The organopolysiloxane elastomers of the present invention are hetero-chain polymers. Preferred organopolysiloxane are ones which are at least partially cross-linked addition reaction products, i.e., hydrosilation products, or addition polymerization products, of an organopolysiloxane having unsaturated groups, such as vinyl or allyl, preferably bonded to at least one terminal silicon atom, and another silicone compound capable of participation in the addition reaction, such as an organohydrogenpolysiloxane. Suitable organopolysiloxane elastomers having a partial three-dimensional cross-linked structure, are described, for example, in US Patent No. 5,266,321, the contents of which are incorporated herein by reference. Examples of dimethicone/vinyl dimethicone crosspolymers are supplied by a variety of suppliers including Dow Coming (DC 9040 and DC 9041), General Electric (SFE 839), Shin Etsu (KSG-15, 16, 18 [dimethicone/phenyl vinyl dimethicone crosspolymer]), Grant Industries (Gransil line of materials), and lauryl dimethicone/vinyl dimethicone crosspolymers supplied by Shin Etsu (e.g., KSG-31, KSG-32, KSG-41, KSG-42, KSG-43, and KSG-44).

The degree of exfoliation can also be controlled by the frequency with which the compositions are applied to the skin and the compositions are applied periodically for a period of time sufficient to exfoliate the skin. Accordingly, the compositions are applied to the skin for a period of at least 2 months, and preferably for at least 4 months, during which time the compositions are applied on a weekly basis. However, a preferred method of obtaining the benefits of the composition is via chronic topical application of the composition to exfoliate the skin. It is suggested, as an example, that "chronic" application be within a range of from about once per week to about 4 to 5 times weekly, preferably daily, most preferably twice daily. By "chronic" application, it is meant herein that the period of,topical application may be over the lifetime of the user, preferably for a period of at least about 6 months to about 20 years, more preferably from about 1 year to about 10 years, and still more preferably from about 2 years to about 5 years, thereby resulting in regular desquamation, which may aid in reducing the appearance of fine lines and wrinkles due to chronological aging or photoaging.

The method of the present invention may include applying in addition to the exfoliating compositions of the present invention, other optional components, depending on the intended additional use of the compositions. These include, but are not limited to, additional exfoliants such as chemical exfoliants and other physical exfoliants, preservatives, fragrances, emollients, antiseptics, anti-inflammatories, antibacterials, stabilizers, antioxidants, vitamins, pigments, dyes, humectants, and propellants, as well as other classes of materials the presence of which in the compositions may be cosmetically, medicinally, or otherwise desired. Such components can be found in the CTFA International Cosmetics Ingredients Dictionary.

While the exfoliating system of the present invention is primarily based on physical exfoliation. The compositions of the present invention can also incorporate chemical exfoliants such as amino sugars that are capable of modulating the adhesion of keratinocytes, e.g., N-acetylglucosamine, N-acetylneuraminic acid and N-acetylgalactosamine. Examples of other additional chemical exfoliants include, but are not limited to, AHAs, for example, lactic acid, or BHAs, for example, salicylic acid. Additional physical exfoliants include but are not limited to, for example, such as pumice, polyethylene, walnut shell powder, ground nuts e.g., almond, and the like, or combinations thereof. The amount of additional exfoliants alone or in combination will depend on the type of exfoliant and the strength of exfoliation desired. Preservatives employed, may be in an amount of from about 0.01 to about 2.00 percent, preferably from about 0.02 to about 1.00 percent, of the formula weight. Examples of suitable preservatives are BHA, BHT, propyl paraben, butyl paraben or methyl paraben or an isomer, homolog, analog or derivative thereof.

For topical application, according to the method of the present invention, the compositions can also be formulated with a variety of cosmetically and/or pharmaceutically acceptable vehicles. Accordingly, the compositions of the present invention comprise a pharmaceutically or cosmetically acceptable carrier, in an amount appropriate to accommodate the other components of the formulation. The term "pharmaceutically and/or cosmetically acceptable vehicle" refers to a base, for either pharmaceutical or cosmetic use, within which the components of the present are contained and which will not cause harm to humans or other recipients. As used herein, "pharmaceutical" or "cosmetic" will be understood to encompass both human and animal pharmaceuticals or cosmetics. There are few limitations on the type of base which is suitable for the compositions containing the heat generating agent encapsulated by a silicone component and the physical exfoliating component. The vehicle may be aqueous, nonaqueous or a combination thereof appropriate for the formulation desired.

The compositions can be prepared in any form convenient for topical application to the skin. Such forms include, but are not limited to creams, lotions, cleansing towelettes, facial masks, creams, dispersions, emulsions (water-in-oil or oil-in-water), suspensions, creams, lotions, gels, foams, mousses and the like. In a preferred embodiment, the carrier is anhydrous, for example, a silicone suspension, dispersion or emulsion, a gelled oil dispersion, or a pressed powder.

The invention is further illustrated by the following non-limiting examples.

### EXAMPLE 1

A composition, according to the present invention is prepared as follows:

### Ingredients

| **Phase I** | |
|---|---|
| Phenyl trimethicone | 5.00 |
| Cyclomethicone/Poly- | |
| Silicone 11/Dimethicone | 30.00 |
| Tween 20 | 5.00 |
| Hydrogenated lecithin | 5.00 |
| Isododecane/polyethylene | 10.00 |

| **Phase II** | |
|---|---|
| Jade Powder | 2.00 |
| Bamboo powder | 0.02 |
| Polyethylene | 3.00 |

| **Phase III** | |
|---|---|
| Encapsulated heat | |
| regulating agent | 34.98 |

| **Phase IV** | |
|---|---|
| Phenyl trimethicone | 5.00 |

To prepare the composition, the materials are combined in the order above by mixing. The composition is topically applied to the skin for exfoliation and heat sensation.

## Claims

1. A method for exfoliation of the skin comprising applying to the skin an anhydrous composition comprising (a) a physical exfoliating system comprising at least three insoluble particles of different types of exfoliating materials having a particle size in the range of between 20 to 500 microns and an average particle size difference between each of at least 50 microns, (b) a heat generating agent chosen in the group consisting of metallic chlorides, bromides, oxides and hydroxides, carbonates and sulfates encapsulated by surface treatment with a silicone and (c) a silicone component as a vehicle for the composition.

2. The method according to claim 1 wherein the smallest particle has a particle size in the range of 20 to 50 microns.

3. The method according to claim 1 wherein the largest particle has a particle size in the range of 300 to 500 microns.

4. The method according to claim 1 wherein the composition is applied weekly.

5. The method according to claim 1 wherein the composition is applied to the skin for a period of at least 2 months.

6. The method according to claim 1 wherein the compositions are applied from 4 to 5 times a week.

7. The method according to claim 6 wherein the composition is applied two times a day.

8. The method according to claim 2 wherein the largest particle is a bamboo extract, the middle-sized particle is polyethylene resin and the smallest particle is jade powder.

9. The method of claim 1 wherein the silicone component is selected from the group of silicone oils, silicone copolymers, silicone elastomers, and combinations thereof.

10. The method of claim 1 wherein the heating agent is encapsulated by a dimethicone.

11. An exfoliant system for topical application to the skin as a heating mask wherein the mask is prepared by a process comprising the step of combining
a) a physical exfoliating system comprising at least three insoluble particles of different types of exfoliating materials having a particle size in the range of between 20 and 500 microns, and an average particle size difference between each of at least 50 microns;
b) an encapsulated heating agent chosen in the group consisting of metallic chlorides, bromides, oxides and hydroxides, carbonates and sulfates encapsulated by surface treatment with a silicone; and
c) a silicone component as a vehicle for the composition.

## Patentansprüche

1. Verfahren zum Abschilfern der Haut, welches die Anwendung einer wasserfreien Zusammensetzung umfasst, die (a) ein physikalisches Abschilferungs-System enthält, das mindestens drei unlösliche Teilchen unterschiedlicher Arten von Abschilferungsmaterialien umfasst, die eine Teilchengröße in einem Bereich von zwischen 20 und 500 Mikrometer und einen Unterschied in der mittleren Teilchengröße von mindestens 50 Mikrometer zwischen den Teilchen aufweisen, (b) ein Wärme erzeugendes Mittel enthält, das ausgewählt ist aus der Gruppe, die aus metallischen Chloriden, Bromiden, Oxiden und Hydroxiden, Karbonaten und Sulfaten besteht, die durch die Oberflächenbehandlung mit einem Silikon eingekapselt sind, und (c) eine Silikonkomponente als Trägersubstanz für die Zusammensetzung enthält.

2. Verfahren nach Anspruch 1, wobei das kleinste Teilchen eine Teilchengröße im Bereich von 20 bis 50 Mikrometer aufweist.

3. Verfahren nach Anspruch 1, wobei das größte Teilchen eine Teilchengröße im Bereich von 300 bis 500 Mikrometer aufweist.

4. Verfahren nach Anspruch 1, wobei die Zusammensetzung wöchentlich aufgetragen wird.

5. Verfahren nach Anspruch 1, wobei die Zusammensetzung für einen Zeitraum von mindestens 2 Monaten auf die Haut aufgetragen wird.

6. Verfahren nach Anspruch 1, wobei die Zusammensetzungen 4- bis 5-mal pro Woche aufgetragen werden.

7. Verfahren nach Anspruch 6, wobei die Zusammensetzung zweimal täglich aufgetragen wird.

8. Verfahren nach Anspruch 2, wobei das größte Teilchen ein Bambusextrakt ist, das Teilchen mittlerer Größe ein Polyethylenharz ist und das kleinste Teilchen ein Jadepulver ist.

9. Verfahren nach Anspruch 1, wobei die Silikonkomponente ausgewählt ist aus der Gruppe, die aus Silikonölen, Silikoncopolymeren, Silikonelastomeren und Kombinationen daraus besteht.

10. Verfahren nach Anspruch 1, wobei das Wärmemittel durch ein Dimethicon eingekapselt ist.

11. Abschilferungs-System zur topischen Aufbringung auf die Haut als eine Wärmemaske, wobei die Maske durch ein Verfahren hergestellt ist, das den Schritt des Kombinierens von
a) einem physikalischen Abschilferungs-System, das mindestens drei unlösliche Teilchen unterschiedlicher Arten von Abschilferungsmaterialien enthält, die eine Teilchengröße in einem Bereich von zwischen 20 und 500 Mikrometer und einen Unterschied in der mittleren Teilchengröße von mindestens 50 Mikrometer zwischen den Teilchen aufweisen;
b) einem eingekapselten Wärmemittel, das ausgewählt ist aus der Gruppe, die aus metallischen Chloriden, Bromiden, Oxiden und Hydroxiden, Karbonaten und Sulfaten besteht, die durch die Oberflächenbehandlung mit einem Silikon eingekapselt sind, und
c) einer Silikonkomponente als Trägersubstanz für die Zusammensetzung umfasst.

## Revendications

1. Un procédé d'exfoliation de la peau comprenant l'application à la peau d'une composition anhydre comprenant
(a) un système d'exfoliation physique comprenant au moins trois particules insolubles de matériaux exfoliants de types différents présentant une dimension de particules comprise entre 20 et 500 microns et une différence de dimensions de particules moyenne entre chaque dimension d'au moins 50 microns,
(b) un agent générateur de chaleur choisi dans le groupe comprenant les chlorures, bromures, oxydes et hydroxydes, carbonates et sulfates métalliques encapsulés par traitement de la surface par un silicone et
(c) un composant à base de silicone constituant un véhicule pour la composition.

2. Le procédé selon la revendication 1, dans lequel la particule de plus petite dimension est formée de particules dont la dimension est comprise entre 20 et 50 microns.

3. Le procédé selon la revendication 1, dans lequel la particule de plus grande dimension est formée de particules dont la dimension est comprise entre 300 et 500 microns.

4. Le procédé selon la revendication 1, dans lequel la composition est appliquée hebdomadairement.

5. Le procédé selon la revendication 1, dans lequel la composition est appliquée à la peau sur une période d'au moins 2 mois.

6. Le procédé selon la revendication 1, dans lequel les compositions sont appliquées à raison de 4 à 5 fois par semaine.

7. Le procédé selon la revendication 6 dans lequel la composition est appliquée à raison de deux fois par jour.

8. Le procédé selon la revendication 2 dans lequel la particule de plus grande dimension est formée d'extraits de bambou, la particule de dimension moyenne est formée de résine polyéthylénique et la particule de plus petite dimension est formée de poudre de jade.

9. Le procédé selon la revendication 1, dans lequel le composant à base de silicone est choisi dans le groupe comprenant les huiles de silicone, les copolymères de silicone, les élastomères de silicone, et leurs combinaisons.

10. Le procédé of revendication 1, dans lequel l'agent générateur de chaleur est encapsulé dans de la diméthicone.

11. Un système exfoliant pour applications topiques à la peau par formation d'un masque chauffant dans lequel le masque est préparé par un procédé comprenant les étapes de combinaison
(a) un système d'exfoliation physique comprenant au moins trois particules insolubles de matériaux exfoliants de types différents présentant une dimension de particules comprise entre 20 et 500 microns et une différence de dimensions de particules moyenne entre chaque dimension d'au moins 50 microns,
(b) un agent générateur de chaleur encapsulé choisi dans le groupe comprenant les chlorures, bromures, oxydes et hydroxydes, carbonates et sulfates métalliques encapsulés par traitement de la surface par un silicone et
(c) un composant à base de silicone constituant un véhicule pour la composition.
